# EUROPEAN PATENT APPLICATION

(11) **EP 1 298 240 A1**
(43) Date of publication of application: **02.04.2003**
(21) Application number: 01938622.6
(22) Date of filing: 13.06.2001
(51) Int. Cl.: D04H 3/16, D04H 3/00, D04H 1/54, A61F 13/514, B32B 5/02

(54) **NONWOVEN-FABRIC LAMINATE AND USE THEREOF**

(30) Priority: 13.06.2000 JP 2000176627
(71) Applicant: Idemitsu Unitech Co., Ltd., Tokyo 112-0002 (JP)
(72) Inventor: ISHIKAWA, Masahide, Kujukuri-machi, Sanbu-gun, Chiba 283-010 (JP); KURAHASHI, Akihiko, Kujukuri-machi, Sanbu-gun, Chiba 283-010 (JP)
(74) Representative: Hucker, Charlotte Jane
(86) International application number: JP0104983
(87) International publication number: WO01096640

(57) **Abstract**

A nonwoven-fabric laminate which comprises a spunbonded nonwoven polyolefin resin fabric having an average fiber diameter of 5 to 60 µm and superposed thereon a permeable water-proofing material (a melt-blown nonwoven-fabric, microporous resin film, etc.) and in which the spunbonded nonwoven-fabric side has a coefficient of static friction of 0.1 to 0.4.

## Description

### Technical Field

The present invention relates to a nonwoven-fabric laminate and use thereof. More specifically, the present invention relates to a nonwoven-fabric laminate comprising a spunbonded nonwoven-fabric consisting of a polyolefin resin which has liquid barrier property, impalpable powder barrier property and gas permeability, especially, good feeling and touch to skin, and excellent strength and post-processability, which is capable of using suitably various uses, particularly to a back sheet material or side gather material for an absorptive article such as disposable diaper or the like, and an absorptive article, a wrapping material for powders and a sheet for medical treatment, which used this nonwoven-fabric laminate.

### Background of the Invention

The spunbonded nonwoven-fabric has been used in many fields, since it has characteristics such as excellent strength, moderate bending rigidity, ductility, gas permeability and the like, and also it has excellent continuous spinability and productivity. As thermoplastic resins used to the spunbonded nonwoven-fabric, which is the long fiber nonwoven-fabric, have been used polyamide resins and polyester resins from melt spinability, fiber characteristic and the like. However polyolefin resins such as polypropylene or polyethylene or the like have been much used to the absorptive article such as the disposable diaper and the like in particular.

The spunbonded nonwoven-fabric comprising these polyolefin resins, for example, the resin of the nonwoven polypropylene resin fabric comprises many resins having different degree of crystallinity, even though homopolymers of propylene, and also it is known nonwoven-fabrics comprising resins having various characteristics such as melting point, strength, elastic modulus and the like by means of copolymers of propylene with ethylene, butene-1 or the like. The nonwoven-fabric used such polypropylene resins has good spinability in the case of resin having high degree of crystallinity, however it has problem of poor flexibility and feeling. And moreover, in the case of polypropylene resins having low degree of crystallinity or low melting point, it has flexibility, however there are problems of large friction resistance of inter fibers, fiber and other metal and the like at the time of spinning, and become very worse in the spinability.

At present, the polypropylene resin nonwoven-fabric used in the absorptive article such as disposable diaper and the like is used much articles made by using resins having around 90 mol % in isotactic pentad fraction, which is indication of crystallinity from strength, spinability, bending rigidity and the like.

However, there are problems that using feel such as flexibility, feeling, touch to the skin and the like of the obtained nonwoven-fabric is not always sufficient in the case of using to the absorptive article such as the disposable diaper, a sanitary napkin, incontinent pad and the like.

This spunbonded nonwoven-fabric comprising the polypropylene resin based long fiber is a material with excellent gas permeability, however, at the time of using as the absorptive material, liquid barrier property is essential requirement, in the case of using as the absorptive article, especially in the back sheet, though depending to the used spot Consequently, to satisfy both characteristic and liquid barrier property of the polypropylene resin based nonwoven-fabric, generally the nonwoven-fabric laminate which is made by forming a composite material with other moisture-permeable water-proofing material is used. And moreover, as to side gather a material made to the composite with a material having good water resistance is used similarly.

However, the absorptive article, which used the nonwoven-fabric laminate, is touched directly to the skin or clothes at the time of using, or hands of workers at the time of attaching or removing. On account of this, it is pointed out that feeling of using or working such as flexibility, feeling, touch to the skin or the like are insufficient, and also feel is plastic like and further it is inadequate to the skin. On account of this, a nonwoven-fabric laminate, which is, improved these characteristics is expected.

On account of this, as to improvement of feeling, touch to the skin or the like as the surface material of these absorptive articles, various proposals are carried out.
(1) A Japanese patent laid open No.88056/1990 proposed to laminated a polyolefin spunbonded nonwoven-fabric having fineness of 1 to 4 denier and area weight of 8 to 28 g/m² and superposed thereon a polyolefin melt brown nonwoven-fabric having fineness of 1 denier or less and area weight of 0.2 to 10 g/m², and contacted both nonwoven-fabrics during these nonwoven-fabrics were presented agglomerating property. However, in the case of the spunbonded fabric having fine diameter of the fiber, the effect is observed, but when the diameter of the fiber become finer, bending rigidity of the spunbonded nonwoven-fabric is lowered, thus working in the process of running, heat sealing or the like in post-processing is not stable, and sometimes high speed and automation of the process become difficult.
(2) A Japanese patent laid open No.143853/1997 proposed a laminated nonwoven-fabric which comprises a composite spunbonded nonwoven-fabric composed of two kind of resins having difference of melting points of 10°C or more and superposed thereon two kind of melt-blown nonwoven-fabrics having difference of melt points of 10°C or more, and fused a resin with a nonwoven-fabric having low melting point. However, the feeling in this case is carried by the melt-blown nonwoven-fabric, the problem of the melt-blown fabric which is characteristic as the surface material of the melt-blown nonwoven-fabric is not dissolved, and also the composite nonwoven-fabric make complex the manufacturing apparatus, and melting points between the composite resins and fluidity are different, thus spinning is difficult in some case.
(3) A Japanese patent laid open No.16115/1998 proposed a moisture-permeable sheet which comprises a gas permeable film and superposed thereon a nonwoven-fabric (spunbonded) having melting point of the same or lower than that of said gas permeable film, and heat fused the both by embossing of emboss pattern having emboss area percentage of 5 to 20 % and no corner part. However, it is considered that combined resins are restricted, and improvement of surface property and feeling are limited due to no special improvement in the spunbonded nonwoven-fabric itself in this case.
(4) A Japanese patent laid open No. 972/1999 proposed a laminated moisture-permeable composite film which comprises a polyolefin biaxially stretched moisture-permeable film and superposed thereon at least one surface a polyolefin based nonwoven-fabric having area weight of 5 to 20 g/m², an average fiber diameter of 0.2 to 2 denier and bulk specific gravity of 0.05 or less. However, this composite film composed mainly of the moisture-permeable stretched film, the nonwoven-fabric itself is special article, which is a special laminate that is not composed mainly of general spunbonded nonwoven-fabric.
(5) A Japanese patent laid open No. 290381/1999 disclosed a back sheet for the absorptive article which comprises a laminate layered alternately layers composed of polypropylene melt-blown nonwoven-fabric and layers composed of polypropylene wet process nonwoven-fabric in which one side surface layer of the laminate comprises the polypropylene wet process nonwoven-fabric layer. That is, by adopting a short fiber wet process nonwoven-fabric, it is intended to secure slipping property of the surface and liquid barrier property. However, the diameter of the fiber of the wet process nonwoven-fabric is relatively finer, there is a problem of causing complex in the manufacturing method.

As described above, the moisture-permeable water-proofing nonwoven-fabric has no performance characteristic such as strength, flexibility, bending rigidity or the like provided to conventional polyolefin resin spunbonded nonwoven-fabric, and hold no characteristic on the production of spinability and low cost, it is only due to making fine diameter of the fiber, adopting of the soft resin, making short fiber, bicomponent spinning and the like.

These improvement technology have the greatest problem which is not maintained adequate bending rigidity necessary to handling without deforming the nonwoven-fabric, in the post processability that is important characteristic to use much the spunbonded nonwoven-fabric into the disposable diaper or the like, that is to say in the process such as sending out, heat adhesion or the like of the nonwoven-fabric. On account of this, it becomes difficult high speed, automatic stable production of the absorptive article. And, especially, from productivity such as spinability or the like, post processability or the like, as to the nonwoven-fabric laminate, it is actual circumstance that the laminate with the polypropylene spunbonded nonwoven-fabric is used still in spite of poor feeling, touch to the skin or the like.

On account of this, it is strongly excepted improvement of these problem afforded to the polypropylene spunbonded nonwoven-fabric from producer and user of the absorptive article such as disposable diaper.

An object of the present invention is to provide a moisture-permeable water-proofing laminate holding substantially characteristic such as strength, bending rigidity, post processability or the like, which the spunbonded nonwoven polypropylene resin fabric has essentially, and also having excellent flexibility, feeling, touch to the skin or the like, a nonwoven-fabric laminate capable of using suitably for the back sheet or the side gather of the absorptive article especially such as the disposable diaper, the sanitary napkin and the like, and the absorptive article, the wrapping material for powder and the sheet for medical treatment used this nonwoven-fabric laminate.

### Disclosure of the Invention

The present inventors investigate wholeheartedly as to the polyolefin resin nonwoven-fabric, especially materials imparted moisture-permeable water-proofing while making the most of function such as gas permeability, flexibility, strength bending rigidity, heat resistance, post processability, automation suitability and the like afforded to the spunbonded nonwoven polypropylene resin fabric, and also feeling of use, which requested to the end product of the nonwoven-fabric especially the absorptive article, such as flexibility, feeling, touch to the skin and the like. As the result, it is found that feeling of use such as flexibility, feeling, touch to the skin and the like can greatly improved, even though in the spunbonded nonwoven-fabric, by controlling its friction characteristic, and the present invention is completed based on this knowledge.

That is, the present invention provides
(1) A nonwoven-fabric laminate which comprises a spunbonded nonwoven polyolefin resin fabric having an average fiber diameter of 5 to 60 µm and superposed thereon a moisture-permeable water-proofing material and in which the spunbonded nonwoven-fabric side has a coefficient of static friction of 0.1 to 0.4.
(2) A nonwoven-fabric laminate according to the above (1), wherein the moisture-permeable water-proofing material is a melt-blown nonwoven-fabric or microporous resin film.
(3) A nonwoven-fabric laminate according to the above described (1 ) or (2), wherein the spunbonded nonwoven-fabric includes a lubricant.
(4) A nonwoven-fabric laminate according to any one of the above described (1) to (3), wherein the melt-blown nonwoven-fabric and microporous film comprises a polyolefin resin.
(5) An absorptive article made by using the nonwoven-fabric laminate according to any one of the above described (1) to (4).
(6) A wrapping material for powder made by using the nonwoven-fabric laminate according to any one of the above described (1) to (4).
(7) A sheet for medical treatment made by using the nonwoven-fabric laminate according to any one of the above described (1) to (4).

### Best Mode to carrying the Invention

In the following description, the present invention will be described in further detail.

A nonwoven-fabric laminate in the present invention is a nonwoven-fabric laminate which comprises a spunbonded nonwoven polyolefin resin fabric having an average fiber diameter of 5 to 60 µm and superposed thereon a moisture-permeable water-proofing material, and in which the spunbonded nonwoven-fabric side has a coefficient of static friction of 0.1 to 0.4.

That is, the nonwoven-fabric laminate is constructed of the polyolefin resin spunbonded nonwoven-fabric on at least one side of the laminate, and nonwoven-fabric on at least one side of the laminate, and in which the coefficient of static friction on the spunbonded nonwoven-fabric side is 0.1 to 0.4. The nonwoven-fabric laminate of the present invention can produce by conjugating in inline or secondarily the polyolefin resin spunbonded nonwoven-fabric having the coefficient of static friction of 0.1 to 0.4 with the moisture-permeable water-proofing material.

The nonwoven-fabric laminate of the present invention comprises basically the spunbonded nonwoven polyolefin resin fabric (A) having the coefficient of static friction of 0.1 to 0.4 and superposed thereon the moisture-permeable water-proofing material (B). Here, as the moisture-permeable water-proofing material is not limited in particular, as far as the material has moisture-permeability, preferably gas permeability, and water proofing property, concretely a melt-blown nonwoven-fabric (B-1) or microporous film (B-2) can exemplify. Moreover, these moisture-permeable water proofing material comprises preferably the polyolefin resin.

In the following description, respective materials constituting nonwoven-fabric laminate of the present invention will be described in further detail.

### (A) Polyolefin Resin Spunbonded Nonwoven-fabric

The spunbonded nonwoven polyolefin resin fabric (A) may be any object having the average fiber diameter of 5 to 60 µm, and satisfying the range of coefficient of 0.1 to 0.4. Consequently, means to control the coefficient of static friction may be optional, and is considered adoption of various means. However, the reason why the nonwoven-fabric laminate of the present invention has excellent flexibility and feeling is considered an effect by improvement of slipping property among long fibers, so that it is preferred a material which improves slipping property of fibers as whole the nonwoven-fabric, as the result, includes a coefficient of static friction as the surface characteristic in a specific range.

The fiber diameter of the fiber composing spunbonded nonwoven polyolefin resin fabric is 5 to 60 µm, preferably 7 to 40 µm, and 7 to 30µm for especially the absorptive article such as the disposable diaper or the like. Here, in the case of 5µm or less, strength and bending rigidity are insufficient, and also in the some case post processability is lowered, and at the same time the using field is greatly limited. Moreover, in the case of 60 µm or more, flexibility is lowered, characteristics as the nonwoven-fabric are loosed, and also in the some case particularly application to the absorptive article, various clothes becomes difficult.

Moreover, area weight of the nonwoven-fabric is in the range of 5 to 200 g/m², preferably 8 to 60 g/m², especially 10 to 30 g/m² for the absorptive article such as the disposable diaper or the like. Here, in the case of area weight of 5 g/m² or less, in the some case strength is insufficient, bending rigidity is lower, and post processability is fallen. Moreover, in the case of 200 g/m² or more, flexibility, gas permeability or the like is lowered, thus characteristics as the nonwoven-fabric is loosen, and in the some case usage becomes difficult due to use.

The characteristic of the nonwoven-fabric of the spunbonded nonwoven-fabric comprising conventional well-known polyolefin resin has the coefficient of static friction of 0.1 to 0.4, preferably 0.12 to 0.35. Here, in the case of the coefficient of static friction is 0.1 or less, processability is lowered by over slippage in the post processability, and at the same time usage amount of additives or surface treatment agent adopted general is necessary due to lowering of the coefficient of static friction, in the some case economic performance is fallen. Further, in the some case heat sealing at the time of post processing, conjugation property by adhesives and the like is trended toward poor, thus such case is not preferred. Moreover, in the case of 0.4 or more, improving effect of feel of usage such as flexibility, feeling, touch to the skin become insufficient.

The spunbonded nonwoven polyolefin resin fabric can combine properly based on the use of the nonwoven-fabric laminate, required property, gas permeability, durability (non-water permeability) or the like.

The coefficient of the nonwoven-fabric laminate of the present invention can determine according to ASTM-D 1894. Concretely, based on measuring conditions of:
Coefficient of static friction measuring machine: made of Toyo Seiki Seisakusho Co. Ltd., A type
Loading plate: 63.6mm×102.2m×19.4mn (height)
Load: 8.87N of iron plate
Incline speed: 2.7 degree/second
sliding velocity θ was determined by piling up measuring surfaces of the nonwoven-fabric, tanθ was obtained, and made it the coefficient of static friction. The smaller the numerical value the better the slipping property.

The nonwoven polyolefin resin fabric used in the present invention is not especially restricted as far as it is the spunbonded nonwoven-fabric, and it can produce by various manufacturing process. Moreover, the bonding form can adopt embossing, calendaring, heat adhesion such as hot air or the like, adhesion by adhesives, mechanical entangling such as needle punching, water punching and the like. However, heat adhesion by embossing is preferred from productivity.

The polyolefin resin used in the spunbonded nonwoven polyolefin resin fabric used in the nonwoven-fabric laminate of the present invention is not especially restricted, it can include a homopolymer of propylene, copolymer of propylene with at least one of α-olefin such as ethylene, butene-1, 4-methyl-pentene-1, hexane-1, octen-1 or the like. These polypropylene resins are selected properly from those that having difference of various crystallinity, molecular weight and distribution of molecular weight from selection of catalysts at the time of polymerization, polymerization condition or the like, based on the property required in the nonwoven-fabric. This selection is examined from the points of strength, bending rigidity, use and the like, but from spinability, vending rigidity, slimy feeling or the like as described above, using of the homopolymer of propylene or the polypropylene copolymer with low ratio of copolymerization is preferred.

Here, crystallinity is selected from a range from 88 to 95 mol %, preferably 89 to 93 mol % in the isotactic pentad fraction in the case of using the nonwoven-fabric as the disposable diaper and the like. Here, the isotactic pentad fraction (IPF) is an isotactic fraction in the pentad unit in the polypropylene molecular chain, which is measured by using nuclear magnetic resonance spectrum (¹³C-NMR) by an isotope carbon for example described in Macromolecules. Vol.28, No.16, PP5403 (1995).

Moreover, melt flow rate (MFR) [according to JIS K 7210, measuring temperature: 230°C, measuring load: 21.18N] of the polypropylene resin is in the range of 5 to 200 g/10 min, preferably 10 to 100 g/10 min. In particular, those that in the range of 30 to 80 g/10 min is suitable for the absorptive material.

Next, polyethylene resins include a homopolymer of ethylene, copolymers of ethylene with α-olefin having 3 to 10 carbon such as propylene, butene-1, 4-methyl-pentene-1, hexane-1, octane-1 or the like, and copolymers of ethylene with a polymerizable monomer such as vinyl acetate, acrylic acid or the like. In particular, the above described ethylene-α-olefin copolymer having density of 880 to 960 kg/m³, preferably 900 to 950 kg/ m³, melting point in the range of 100 to 140°C, preferably 110 to 130°C, and melt flow rate (MFR) [according to JIS K 7210, measuring temperature : 190°C, measuring load : 21.18N] of 5 to 60 g/10 min, preferably 10 to 50 g/10 min is used preferably from points of spinability, melting point, bending rigidity and the like.

Moreover, these polypropylene resins and polyethylene resins may be mixture of two kinds or more respectively, and can use as resin compositions containing other ethylene resin, propylene resin, thermoplastic elastomer or the like of less than 50 mass %.

Next, means to obtain the spunbonded nonwoven-fabric having the coefficient of static friction in the range of 0.1 to 0.4 are not restricted especially, and various means are included. Concretely, it can exemplify roughly divided to (1) melt spinning process by combining a lubricant into a polyolefin resin for spinning, and (2) surface treating process to the fiber after spinning.

Here, the lubricant is not especially restricted, and include fatty acid amide compound, fatty acid compound, paraffin and hydrocarbon resin, silicone compound, silicone polymer, fluorine compound, fluorine polymer such as copolymer of tetrafluoroethylene with propylene, copolymer of vinylidene fluoride with hexafluoroethylene or the like, or these mixture. Among them the fatty acid amide compound is preferably used.

The fatty acid amide compound include fatty acid monoamide compound, fatty acid diamide compound, saturated fatty acid monoamide compound, unsaturated fatty acid diamide compound. Concretely, it includes amide laurate, amide myristate, amide palmitate, amide stearate, amide behenate, amide oleate, amide erucate, amide montanate, amide N,N'-methylene-bis-laurate, amide N,N'-methylene-bis-myristate, amide N,N'-methylene-bis-palmitate, amide N,N'-methylene-bis-behenate, amide N,N'-methylene-bis-oleate, amide N,N'-methylene-bis-erucate, amide N,N'-ethylene-bis-oleate, amide N,N'-ethylene-bis-erucate and the like, and also these can be used by combining plural kind of substances.

Among these fatty acid amide compounds, amide erucate, which is an unsaturated fatty acid monoamide compound, is preferably used. The reason why it is used is they are suitable to decrease the coefficient of static friction of the nonwoven-fabric, by decreasing of spinability by exposing unnecessarily the fatty acid amide on the surface at the time of spinning of the fiber of the nonwoven-fabric, and ageing of the nonwoven-fabric containing the fatty acid amide compound described below. Content of the fatty acid amide compound in the polyolefin resin is in the range of 0.05 to 1 mass %, preferably 0.1 to 0.5 mass %. This content is decided by judging synthetically kind of the polyolefin resin, the resin characteristics such as crystallinity, MFR and the like, kind of the fatty acid amide compound, required property of the obtained nonwoven-fabric, ageing condition and the like.

Consequently, for example, in the case of using amide erucate in the homopolymer of propylene having isotactic pentad fraction of around 90 mol %, it is preferred in the range of 0.1 to 0.5 mass %, especially 0.2 to 0.4 mass %. In this case, although it depends on ageing treatment condition, if it is 0.2 mass % or less, in some cases it is difficult to control the coefficient of static friction of the nonwoven-fabric in the range of 0.1 to 0.4, and if it is 0.4 mass % or more, in some cases the amount of the amide erucate on the surface of the nonwoven-fabric is much, it becomes cause of worse of appearance such as generation of white powder or the like, or lowering of heat fusion property and post processability.

Further, the polyolefin resin can be added well-known additives component used generally in the nonwoven-fabric for use or imparting characteristic or the like of the nonwoven-fabric laminate. These well known additives components include neutralizating agents such as calcium stearate, hydrotalcite or the like, antioxidants such as phenol based, phosphorus based, sulfur based or the like, heat stabilizers, nucleus forming agents, UV absorbers, light stabilizers, antistatic agents, flame retardants, pigments, dyes, or inorganic powders such as silica, talc, calcium carbonate, calcium oxide, magnesium oxide or the like.

The manufacturing method of one example of the nonwoven-fabric composing the present invention make a spunbonded primary nonwoven-fabric by melt spinning a mixture which is dry blended fixed amount of lubricant such as fatty acid amide or the like and additives component added as the need arises to a polyolefin resin.

Here, the spunbonded nonwoven-fabric can be obtained for example, a primary nonwoven-fabric well known method which comprises melt extruding the raw material polyolefin resin having the above described combination, spinning it from a spinneret for spinning, taking up the spun fiber with an airborne tracking apparatus such as air sucker or the like, opening as the need arises, collecting fibers with air flow by a web collecting apparatus such as a net conveyer or the like, partial fusing with heating means such as heated air, heating roll or the like, especially a heating roll as the occasion demands, thereafter winding up.

Further, this spunbonded nonwoven polyolefin resin fabric is a nonwoven-fabric comprising ordinarily polyolefin resin alone, but it may be a composite fiber nonwoven-fabric comprising a polyolefin resin of at least 50 % on the surface of the fiber.

These composite fiber nonwoven-fabric may be used the composite fiber having core-sheath structure composing of a polyolefin resin as the sheath component and a resin having higher melting point than the sheath component resin excepting the polyolefin resin such as a polyamide resin, polyester resin or the like as the core component, or the composite fiber having side by side structure which comprises the polyolefin resin of ordinarily 50 mass % or more of fibers and the other resin in the rest. Further, this core-sheath structure composite fiber and side-by-side structure composite fiber may be, of course, combination of two kind of different polyolefin resins among polyolefin resins.

Thus obtained primary nonwoven-fabric is excellent in spinability, however in some cases the coefficient of static friction specified by the nonwoven-fabric of the present invention is not expressed by itself, by the kind of the lubricant, especially in the case of the fatty acid amide compound. In this case, to control the coefficient of static friction of the nonwoven-fabric in the range of 0.1 to 0.4, by only ageing this primary nonwoven-fabric under heating, it can be the range of the coefficient of static friction, which is specified by the present invention. In the conventional nonwoven-fabric manufacturing apparatus, such ageing apparatus is not inserted, generally ageing was not performed.

Here, ageing condition is differed due to kind of the polyolefin resin, resin characteristic such as degree of crystallinity, density, melting point or the like, kind and melting point of contained the fatty acid amide compound and solubility to the polyolefin resin or the like. Consequently, by considering characteristic of the polyolefin resin which is the raw material of the nonwoven-fabric, or characteristic of the fatty acid amide compound as the lubricant, and by considering the range of the coefficient of static friction in the range of 0.1 to 0.4, required characteristic such as flexibility, feeling, touch to the skin or the like required to the end product, concretely the treating condition is decided experimentally.

For example, the spunbonded nonwoven polyolefin resin fabric used in the present invention is obtained by ageing a spunbonded nonwoven-fabric made by melt spinning the polyolefin resin which containing the fatty acid amide compound at a temperature of 30 to 60°C for around 1 to 50 hours.

For example, in the homopolymer of polypropylene, as a concrete example in the case of that having isotactic pentad fraction of around 90 mol % and content of amide erucate of 0.3 mass %, the following ageing treatment condition can set up.

In the case of the ageing temperature of 40°C, the ageing time is 5 to 50 hours, preferably about 8 to 12 hours. Moreover, in the case of the ageing time is 24 hours, the ageing temperature is 32 to 50°C, preferably about 33 to 40°C. When the ageing condition is milder than the range described above, lowering of the coefficient of static friction requires longer time, in some cases productivity is lowered. Further, when the ageing condition is severer than the above described range, in some cases the coefficient of static friction become higher reversely, thus it is not preferred.

This ageing treatment can ordinarily carried out in an ageing room which heated air is circulated, by arranging core pipes in the state of winding the nonwoven-fabric in roll form. When this ageing is performed, even though the nonwoven-fabric is wound state in roll form, the nonwoven-fabric is obtained approximate uniform effect of ageing treatment due to gas permeability of the nonwoven-fabric. Further, ageing can also carried out by roll heating and/or heated air, the nonwoven-fabric is not wound state, but while it is running between rolls.

Next, the other method to give the coefficient of static friction of the nonwoven-fabric composing the laminated nonwoven-fabric of the present invention is a method by surface treatment of the fiber of the nonwoven-fabric obtained by spinning. As the surface treating agent, for example, dimethyl siloxane, methyl hydrogen polysiloxane, compound containing fatty acid amide can be used. However, this surface treatment has problem that cannot treat into inner pert in some cases, due to wet treating process, drying process and thickness of the nonwoven-fabric. Therefore, in many cases the method by melts blending of the lubricant described above is preferred, due to form, use, and range of the coefficient of static friction or the like of the nonwoven-fabric.

The spunbonded nonwoven-fabric comprising the polyolefin resin composing the nonwoven-fabric laminate of the present is generally hydrophilicity, owing to use of the absorptive articles and the like, for example, in the case of using as the top material of the disposable diaper or sanitary napkin or the like, in some cases hydrophilicity of only water permeable is required. In this case, the nonwoven-fabric can do hydrophilicity imparting treatment.

This hydrophilicity imparting treatment include introduction of hydrophilic groups such as carboxyl group or the like by ozone treatment, or surface treatment by hydrophilic compounds, however treatment by solution of hydrophilic compounds is preferred in the point of the effect. As the imparting treatment method, spraying method, coating method, immersing method and the like can be exemplified. Moreover, the hydrophilic compound can be exemplified, for example, alkyl-ester such as polyalcohol having 8 to 26 carbon atoms containing polyoxyethylene, alkyl-ether, polyether containing fatty acid amide group, fatty acid monoglyceride, sorbitol ester derivative, alkyl-phosphate metal salt, alkyl-sulfate metal salt, polyoxyethylene alkyl ether sulfate metal salt, alkyl sulfosuccinate metal salt, sugar derivative having glucose ring and the like.

The spunbonded nonwoven-fabric composed of the polyolefin resin is maintained essential characteristic of the nonwoven-fabric which has the polyolefin resin itself, that is, strength, heat resistance, bending rigidity and the like as they are, by lowering of the coefficient of static friction by the ageing treatment or the surface treatment, and flexibility, feeling, touching feel such as touch to the skin, and usage feel are improved markedly.

Next, the other component of the present invention will be described.

### (B) Moisture-Permeable Water-proofing Material

The moisture-permeable water proofing material can be used well known material itself. Firstly, preferable material is explained concretely.

### (B-1) Melt-blown Nonwoven-fabric

The melt-blown nonwoven-fabric is a nonwoven-fabric comprising very fine fiber, ordinarily which comprises having the fiber diameter of 0.1 to 10 µm, preferably 0.2 to 6 µm, and having area weight of 1 to 100 g/m², preferably 1 to 50 g/m². These fiber diameter and area weight has great influence on moisture-permeability, gas permeability, water-proofing, leakage resistance to impalpable powder and the like, so as to satisfy characteristic required by use of the nonwoven-fabric laminate, these value can select properly from range described above. For example, area weight is 1 to 5 g/m² in the case of the diaper side gather, and is 10 to 50 g/m² in the case of the diaper back sheet or sheet for medical treatment.

The melt-blown nonwoven-fabric can be obtained by well-known technique which is a method composed of jetting heated high speed air stream against fine flow of molten thermoplastic resin, forming very fine fiber by drawing the molten resin by action of the air stream, and forming a sheet by collecting.

The thermoplastic resin composing this melt-blown nonwoven-fabric is used crystalline resins such as polyolefin resin such as polypropylene resin, polyethylene resin or the like, polyester resin such as polyethylene terephthalate and the like, polyamide resin such as nylon 6, nylon 66 and the like. Among them, the polyolefin resin, especially polypropylene resin is used preferably from melt-blown spinability, water-proofing property and the like.

### (B-2) Microporous Film

The microporous film is not especially restricted, as far as it is films containing many micropores for gas permeability. As the microporous film, the polyolefin resin microporous film is used preferably. The manufacturing method of the microporous film is optionally, and well-known films are used. The polyolefin resin microporous film include, for example, a film obtained by a method which forming micropore structure by eluting filler or plasticizer with a solvent from a film composed of a polyolefin resin containing inorganic filler, organic filler or plasticizer or the like, and a film obtained by at least uniaxial drawing a film composed of a polyolefin resin containing inorganic filler or organic filler, and the like.

Among them, the latter film obtained by at least uniaxial drawing the film composed of the polyolefin resin containing inorganic filler or organic filler is preferably used. The polyolefin resin used in the present invention is high density polyethylene, medium density polyethylene, ethylene-α-olefin copolymer, high-pressure process low density branched polyethylene, polypropylene, copolymer of propylene with the other olefin, or mixture of these polyolefin. Among them, polyethylene resin is preferred owing to use from the view point of flexibility in some cases.

This polyethylene resin has density in the range of ordinarily 880 to 960 kg/m³, preferably 900 to 950 kg/m³, and melt flow rate (MFR) [according to JIS K 7210, measuring temperature: 190°C, measuring load: 21.18N] in the range of ordinarily 0.01 to 10 g/10 min, preferably 0.02 to 5 g/10 min.

The filler containing in the polyolefin resin is used inorganic or organic filler, for example, inorganic filler such as calcium carbonate, talc, clay, kaolin, silica, diatomaceous earth, magnesium carbonate, barium carbonate, barium sulfate, calcium sulfate, calcium sulfite, calcium hydroxide, magnesium hydroxide, aluminum hydroxide, zinc oxide, calcium oxide, magnesium oxide, titanium oxide, mica, alumina, zeorite, glass powder and the like, organic filler such as wood flour, cellulose powder, high melting temperature resin powder, crosslinked resin powder and the like is used.

The average particle diameter of these filler is in the range of ordinarily 30 µm or less, preferably 0.2 to 10 µm. Here, in the case of too small in the particle diameter, dispersibility and formability are worse, and in the case of too large, precision of the micropore of the drawn film becomes worse, and water-proofing property (water pressure resisting property) is lowered in some cases. These can also be contained plural from necessity. Moreover, these filler can also be used those that surface treated with the fatty acid or the fatty acid metal salt or the like.

Here, content of the inorganic filler is 20 to 400 mass parts, preferably 40 to 300 mass parts relative to the polyolefin resin of 100 mass parts. Here, when content of the filler is 20 mass % or less, forming of micropore in the case of drawing the film is insufficient, and moisture-permeability is not secured sufficiently. Moreover, if content over 400 mass parts, kneading property, dispersibility, and film forming property is lowered, and at the same time strength is also decreased in some cases.

Further, from necessity, other resin, elastomer and various additives, which are used regularly in the polyolefin resin film containing the filler, can be also combined. For example, ethylene-propylene copolymeric elastomer, liquid or solid hydrocarbon resin, liquid polybutadiene containing active hydrogen, plasticizer, radical generator, thermo stabilizer, UV absorber, higher fatty acid, lubricant such as its ester, its amide, its metal salt, coloring agent, and flame retardant can be exemplified.

The polyolefin resin is pellelized together with fixed amount of filler and various additives by using Banbury mixer, kneading extruding making machine. By using this pellet and T die extruder and inflation molding machine, a film is formed. The formed film is drawn approximate 1.5 to 10 times in at least uniaxial direction. Drawing may be performed in multi-stage, and also biaxial drawing.

Drawing of the film is performed in the range of temperature from lower 100°C than melting point of the polyolefin resin to lower 20°C than the melting point. By this drawing, strength of the film is improved and at the same time micropores are formed. By heat treating this drawn film, dimensional precision of the film can be improved. Further, surface treatment to improve adhesive property such as corona treatment, flame treatment and the like can also be performed against the laminating side of the film. Thickness of thus obtained polyolefin resin microporous film is in the range of 10 to 200 µm, preferably 15 to 100µm. It is desirable that having micropores of average diameter of 0.1 to 50 µm, and void fraction of approximate 10 to 80 %.

Such polyolefin resin microporous film itself is well known, and various films can available from the market. For example, the polyethylene drawn moisture-permeable film can include "Polam" made of Tokuyama Co. Ltd., "Breslon" made of Nitto Denko Co. Ltd., and "Espowal" made of Mitsui Kagaku Co. Ltd.

Moisture-permeability and water-proofing property of moisture-permeable water-proofing material described above in detail is difficult to determine owing to use which the nonwoven-fabric laminate of the present invention is used, but generally degree of moisture-permeability is usually 1,000 g/m²·24 hours or more, preferably 2,000 g/m²·24 hours or more. Moreover, water-proofing property (water pressure resisting property) is different due to use, however it is 1 k Pa or more, preferably 10 k Pa or more. Consequently, in the case of required water-proofing property, usage of the microporous film is preferred. For example, laminate with the microporous film is used for the back sheet of hips of the disposable diaper from water permeability, feel of usage and the like, and laminate with the melt-blown nonwoven-fabric is used for the side gather. Further, the measuring method will be described after. To satisfy these characteristics, it can decide by selection of the fiber diameter and area weight of the melt-blown nonwoven-fabric, or size and number of the micropore, thickness of the microporous film, owing to use of the nonwoven-fabric laminate, and based on the required characteristics.

### (On Lamination)

The nonwoven-fabric laminate of the present invention comprises the laminate structure of specific spunbonded nonwoven-fabric with the moisture-permeable water-proofing material described above. Moreover, the moisture-permeable water-proofing material may be other material except described above. The laminating method of the both materials is not especially restricted, various methods can adopt.

As to laminating method, usually the above described, for example, spunbonded nonwoven polyolefin resin fabric, which is ageing treated and has the coefficient of static friction of 0.1 to 0.4, is laminated and adhered with the moisture-permeable water proofing material so as to be come the nonwoven-fabric on at least one side. However, according to circumstances, the spunbonded nonwoven-fabric is laminated with the melt-blown nonwoven-fabric or the microporous film or the like, thereafter they can be also ageing treated. In this case, for example, an inline laminating method in which when the melt-blown nonwoven-fabric is spun on the spunbonded nonwoven-fabric, and from necessity further spunbonded nonwoven-fabric is manufactured in multi-step and continuously thereon, a multi-layer nonwoven-fabric is previously manufactured by containing fatty acid amide into a polyolefin resin for at least one side spunbonded nonwoven-fabric, and then this multi-layer nonwoven-fabric is ageing treated is given.

Further, as the laminating means to form this nonwoven-fabric laminate, various laminating means such as heat adhesion, adhesion with adhesives and the like is given, although it is different by the moisture-permeable water-proofing material. However, in the case of the melt-blown nonwoven-fabric, simple and low cost above described inline laminate heat adhesion means, especially heat embossing roll method can be adopted. This embossing roll method can laminate by using well-known laminating apparatus with an embossing roll and a flat roll. Here, the embossing roll can adopt various shape embossing pattern, there are lattice state in which each fused part is continued, independent lattice state, optional distribution and the like. Moreover, emboss area fraction is in the range of approximate 5 to 40 %.

Moreover, in the case of laminating with the moisture permeable water proofing film, heat embossing roll method, dry laminating method or the like can adopt The heat embossing roll lamination condition is different by melting point of the spunbonded nonwoven-fabric or the polyolefin resin micro porous film, and make either layer the emboss side, the condition is selected properly by taking respective factor into consideration. These emboss pattern, emboss area fraction, temperature, pressure and the like can be properly selected depending on the fiber diameter, thickness, area weight, gas permeability, processing speed of the nonwoven-fabric, and further melting point, thickness and the like of other microporous film or the like.

Furthermore, the dry laminating method is important that moisture permeability and gas permeability as the nonwoven-fabric laminate of the present invention is necessary to secure over certain level, and to control by coating amount of the hot melt adhesives to coat on the laminating side, ratio of abundance of the fused point and the like. Concretely, amount of the hot melt adhesives is 0.1 to 5 g/m², and as the coating method, it is not general coating but adoption of means which can partly adhere by jetting to fiber state is preferred. The adhesive in this case is used ethylene-vinyl acetate based, polyurethane based, polyester based adhesives and the like. Moreover, a method to do dry laminate by coating the adhesives in pattern state can be also adopted.

The nonwoven-fabric laminate of the present invention can be made nonwoven-fabric laminates having various characteristics by selection and combination of respective kind, area weight, thickness, moisture permeability and the like of the spunbonded nonwoven polyolefin resin fabric and the moisture-permeable water-proofing material. Consequently, the laminate is possible to develop into various uses by having moisture (gas) permeability, water proofing property, impalpable powder permeable resistance, and also excellent characteristics such as strength, bending rigidity, flexibility, feeling, touch to the skin and the like and excellent bending rigidity. Concretely, the laminate is used effectively in the back sheet or side gather of the absorptive article (sanitary article) such as disposable diaper, sanitary napkin, incontinence pad and the like, sheet material for wrapping of (functional) powder, sheet for medical treatment.

### (Examples)

In the following, the present invention will be illustrated in further detail based on manufacturing examples. However, the present invention is not limited by these manufacturing examples.

### [Manufacturing Example 1: Inline manufacture of a nonwoven-fabric laminate]

As a nonwoven-fabric manufacturing apparatus, a multi-step apparatus was used. To a crystalline polypropylene resin [isotactic pentad fraction: 91 mol %, MFR: 60 g/10 min, melting point: 160°C] 100 mass parts, a phenol based antioxidant (made of Ciba Special Chemical Company, lrganox 1010):0.035 mass parts, phosphorus based antioxidant (made of Sando Company, Sandostab P-EPQ):0.035 mass parts, a neutralizer (made of Kyodo Yakuhin Co. Ltd., calcium stearate) 0.025 mass parts and amide erucate : 0.03 mass parts were dry blended with a super-mixer, thereafter melt kneading at 220°C by using 65 mm ϕ extruder, and melt spun by extruding from the spinneret. The spinneret in this case was diameter of hole of the spinneret: 0.3 mm, and numbers of it in width direction: 200, and extrusion direction: 15.

Then, the spun fiber group were introduced into an air sucker and tracked and drawn, and collected on a belt having a suction apparatus. On this spunbonded nonwoven polypropylene fabric, a melt-blown nonwoven-fabric was formed with a polypropylene resin which is the above described crystalline polypropylene resin containing no amide erucate, thereafter further on it the same spunbonded nonwoven polypropylene fabric as described above was laminated, and then it was sent to heated embossing rolls [an embossing roll at 140°C/a flat roll at 140°C] and emboss-adhered, and rolled it onto a paper tube, thus a primary nonwoven-fabric laminate composed of three layers of the spunbonded nonwoven-fabric/the melt-blown nonwoven-fabric/the spunbonded nonwoven-fabric was obtained.

The obtained rolled nonwoven-fabric laminate was ageing treated in an ageing condition [temperature: 40°C, time: 24 hours] to obtain three layers nonwoven-fabric laminate of the present invention. End breakage or jogging in the spinning process was not observed, and it showed good spinability. The obtained nonwoven-fabric laminate was three layers nonwoven-fabric laminate of spunbonded nonwoven-fabrics [an average fiber diameter: 18 µm,area weight: 7 g/m^{2]}, a melt-blown nonwoven-fabric [an average fiber diameter: 1 µm,area weight: 3 g/m²]. This three layers nonwoven-fabric laminate showed a coefficient of static friction between the spunbonded sides: 0.25, degree of bending rigidity: 5.5 cm, touch to the skin: ⓞ, feeling: ⓞ. And, ⓞ shows excellent, ○ good, and Δ insufficient. Moreover, the laminate had moisture permeability : 8,000 g/m²·24 hours, water pressure resistance : 2 k Pa.

Further, evaluation of spinability and the nonwoven-fabric laminate was performed based on the following description.

### (1) Coefficient of Static Friction

Measurement was performed according to the measuring method of the coefficient of static friction of ASTM-D 1894. And, the detail was described above.

### (2) Degree of Bending Rigidity

Measurement was performed according to JIS L 1096 (45°cantilever method).

### (3) Touch to the skin feeling

A functional test by touch to the skin·handle by 20 monitors was performed, and evaluation of ⓞ, ○ and Δ was performed. And, the functional test was performed on the three layers laminate used general spunbonded nonwoven-fabric containing no amide erucate as Δ.

### (4) Moisture Permeability

Measurement was performed according to JIS Z 0208 (cap method).

### (5) Water Pressure Resistance

Measurement was performed according to JIS L 1092 (high water pressure method).

### [Manufacturing Example 2; thermal lamination]

A single layer nonwoven-fabric was obtained by using the single step nonwoven-fabric manufacturing apparatus according to manufacture of the spunbonded nonwoven-fabric containing amide erucate in the front step in the manufacturing example 1 described above, and then ageing treatment was performed, and a spunbonded nonwoven-fabric having an average fiber diameter: 20 µm, area weight: 15 g/m², melting point: 160°C made of a polypropylene resin was obtained. A melt-blown nonwoven polypropylene fabric [Microlex] PC 0020 [An average fiber diameter: about 1 µm, area weight: 20 g/m², melting point: 158°C] made of Kuraray Co. Ltd. was sandwiched between two these spunbonded nonwoven-fabric, and heat laminated by putting the melt-blown nonwoven-fabric into the middle, by using a heat laminating apparatus [a lamimachine made of Sansei Seiki Company, oil temperature control, roll diameter: 300 mm, embossing roll/smooth roll: emboss press bonding percentage: 21%, check pattern (pitch: 2 mm), roll setting temperature : 160°C, embossing pressure : 300 N/cm, lami-speed : 10 m/min], and good three layers nonwoven-fabric laminate was obtained. As a result of evaluation according to the example 1, this three layers nonwoven-fabric had a coefficient of static friction between spunbonded sides: 0.22, degree of bending rigidity: 6.0 cm, touch to the skin: ⓞ, feeling: ○. And also, the laminate had moisture permeability : 6,000 g/ m²- 24 hours, water pressure resistance: 5 k Pa.

### [Manufacturing Example 3: Lamination with Microporous Film]

According to the manufacturing example 2, manufacture of a spunbonded nonwoven-fabric containing amide erucate, and ageing treatment were performed, and a spunbonded nonwoven-fabric having an average fiber diameter: 16 µm, area weight: 20 g/m², melting point: 160°C made of polypropylene resin was obtained.

And then, to this spunbonded nonwoven polypropylene fabric, ethylene-vinyl acetate based hot melt type adhesives [H-6805] [made of Nitta Findlay Co. Ltd.] was sprayed so as to be coating amount of 4g/m² in fibrous state by using a nozzle discharge system fiber spray die. And then a drawn PE moisture permeable film containing inorganic filler made of Tokuyama Co. Ltd. [Pouram PU 35] (thickness 35 µm) was laminated thereon, and a nonwoven-fabric laminate was obtained. As a result of evaluation according to the example 1, this nonwoven-fabric laminate had a coefficient of static friction between spunbonded sides: 0.18, degree of bending rigidity: 5.8 cm, touch to the skin: ⓞ, feeling: ⓞ. And also, the laminate had moisture permeability : 4,500 g/m²·24, hours, water pressure resistance : 100 k Pa.

### Industrial Availability

The present invention is usable to the nonwoven-fabric laminate and the absorptive article, the wrapping material for powder and the sheet for medical treatment used this nonwoven-fabric laminate, and especially it is usable suitably as the back sheet material or side gather material for the absorptive article such as disposable diaper and the like.

## Claims

1. A nonwoven-fabric laminate which comprises a spunbonded nonwoven polyolefin resin fabric having an average fiber diameter of 5 to 60 µm and superposed thereon a moisture-permeable water-proofing material and in which the spunbonded nonwoven-fabric side has a coefficient of static friction of 0.1 to 0.4.

2. A nonwoven-fabric laminate according to claim 1, wherein the moisture-permeable water-proofing material is a melt-blown nonwoven-fabric or microporous resin film.

3. A nonwoven-fabric laminate according to claim 1 or 2, wherein the spunbonded nonwoven-fabric includes a lubricant.

4. A nonwoven-fabric laminate according to any one of claims 1 to 3, wherein the melt-blown nonwoven-fabric and microporous film comprises a polyolefin resin.

5. An absorptive article made by using the nonwoven-fabric laminate according to any one of claims 1 to 4.

6. A wrapping material for powder made by using the nonwoven-fabric laminate according to any one of claims 1 to 4.

7. A sheet for medical treatment made by using the nonwoven-fabric laminate according to any one of claims 1 to 4.
